# EUROPEAN PATENT SPECIFICATION

(11) **EP 3 932 352 B1**
(45) Date of publication and mention of the grant of the patent: **18.11.2026**
(21) Application number: 20831470.8
(22) Date of filing: 22.06.2020
(51) Int. Cl.: A61B 18/14, A61B 18/12, A61B 18/00, A61B 17/00

(54) **HIGH-FREQUENCY TREATMENT DEVICE**
HOCHFREQUENZBEHANDLUNGSVORRICHTUNG
DISPOSITIF DE TRAITEMENT À HAUTE FRÉQUENCE

(30) Priority: 25.06.2019 JP 2019117879
(43) Date of publication of application: 05.01.2022
(73) Proprietor: Kabushiki Kaisha Top, Adachi-ku, Tokyo 120-0035 (JP)
(72) Inventor: IKEUCHI, Atsushi, Tokyo 120-0035 (JP)
(74) Representative: Schön, Christoph
(86) International application number: PCT/JP2020/024321
(87) International publication number: WO 2020/262279

(56) References cited:
- JP-A- 2012 516 724
- JP-A- 2013 521 914
- US-A1- 2006 251 929
- US-A1- 2011 306 960
- US-A1- 2013 012 937
- US-A1- 2016 199 116
- US-A1- 2017 049 513
- US-A1- 2017 049 513

## Description

### Technical Field

The present invention relates to a high-frequency (radiofrequency) treatment device for applying a high-frequency electric current to an object to be treated such as a human or an animal to perform treatment such as thermocoagulation of neural tissue or ablation of tumor tissue.

### Background Art

Conventionally, in the medical field, high-frequency treatment has been widely used by arranging an electrode in the body such as a human or an animal to make a high-frequency electric current flow from this electrode in order to perform tissue ablation. Further, in recent years, a technique for performing a nerve block by high-frequency treatment has been attracting attention as one pain treatment. The nerve block by the high-frequency treatment has the advantages that there are fewer side effects on surrounding tissue and that the effect lasts for a longer time than a conventional method of using drugs.

There are two methods of doing this nerve block by the high-frequency treatment: radiofrequency thermocoagulation and pulsed radiofrequency. The radiofrequency thermocoagulation as one of the two methods is to heat part of nerve tissue at a temperature of about 80°C for a few minutes by a high-frequency electric current flowing from an electrode so as to cause thermocoagulation of the part of nerve tissue in order to block the pain signal, and the pulsed radiofrequency as the other of the two methods is to heat part of nerve tissue at a temperature of 42 °C or lower for ten and several minutes by making a high-frequency electric current flow intermittently into the part of nerve tissue in order to block the pain signal without damaging the nerve.

In such a nerve block, there is a case where multiple nerves are heated together in addition to heating part of one nerve in a pinpoint manner. For example, when a nerve block for sacroiliac joint pain is performed by high-frequency treatment, there is a need to apply heat over a wide area of lateral branches of the dorsal rami of sacral nerves (for example, see Patent Literature 1).

Therefore, in the conventional technique, plural needles are punctured near the lateral branches and electrodes are inserted into the needles as illustrated in FIG. 1 and FIG. 2 of Patent Literature 1 to perform treatment by making a high-frequency electric current flow between these electrodes and a dispersive electrode placed on the skin surface of a patient (so-called monopolar) or by making the high-frequency electric current flow between adjacent two electrodes (so-called bipolar) as illustrated in FIG. 5 of Patent Literature 1.

However, since only one electrode is used for one treatment in the monopolar and an area capable of being heated by one electrode is narrow, when heating of a wide area is required, there is a need to perform treatment plural times by replacing the electrode with any other electrode, causing a problem of requiring time and effort. Further, since heat is applied to an area between and around two electrodes in the bipolar, an area wider than that in the monopolar can be heated by one treatment. However, when a nerve block is performed for sacroiliac joint pain, there is also a need to perform treatment plural times by replacing the electrodes.

In contrast, in the technique proposed in Patent Literature 1, an arcuate electrode is provided at the tip of each helical needle and arranged along a posterior sacral foramen to be able to heat a relatively wide area of the lateral branch of the dorsal ramus of sacral nerve evenly by one treatment.
US 2017/049513 A1 discloses a system and a method for applying radiofrequency electrical energy to a living body, wherein said system for applying electrical energy to body tissue comprises at least three electrodes, each of the at least three electrodes having an exposed conductive tip and a temperature sensor in the conductive tip, each electrode being adapted to be inserted into a patient's body so that said conductive tip will contact the tissue in the patient's body and the temperature sensor will sense the temperature of the tissue near said conductive tip, a generator that produces a high-frequency signal output between at least two output poles, a temperature detector that measures the temperatures from the temperature sensors, a switching system by which each of the at least three electrodes can be connected to and disconnected from each of the at least two output poles, and a controller that automatically produces a sequence of steps to regulate at the same time the temperatures measured from all of the at least three electrodes.

### Citation List

### Patent Literature

Patent Literature 1: Japanese Translation of PCT International Application Publication No. 2018-511444

### Summary of Invention

### Technical Problem

However, although the technique proposed in Patent Literature 1 solves the problem of uneven heating when a heating area is wide, there is also a need to perform treatment three times by rearranging electrodes for each of posterior sacral foramens S1 to S3. Further, there is also a problem of requiring time and effort to insert each helical needle more than to insert a straight needle.

In view of such circumstances, the present disclosure is to provide a high-frequency treatment device capable of heating a wide area efficiently.

### Solution to Problem

A high-frequency treatment device according to the present invention is defined in appended claim 1. Preferred embodiments are defined in the appended dependent claims.

### Advantageous Effects of Invention

According to the high-frequency treatment device of the present invention, there is an excellent advantage that a wide area can be heated efficiently.

### Brief Description of Drawings

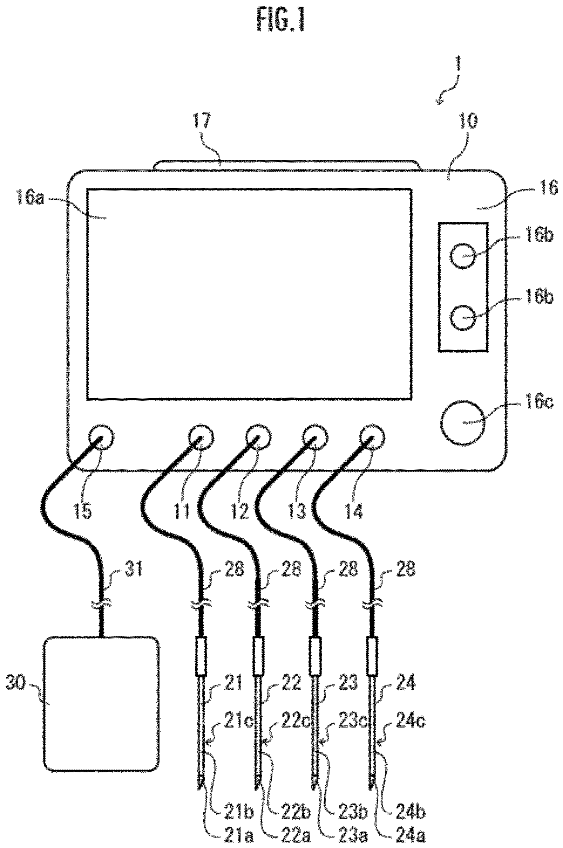

FIG. 1 is a schematic diagram illustrating the appearance of a high-frequency treatment device.

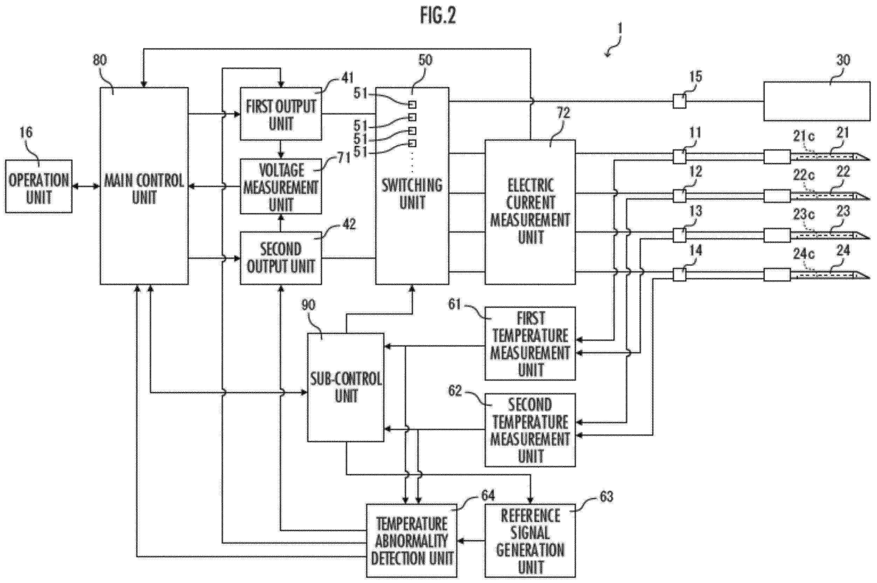

FIG. 2 is a block diagram illustrating the internal configuration of the high-frequency treatment device.

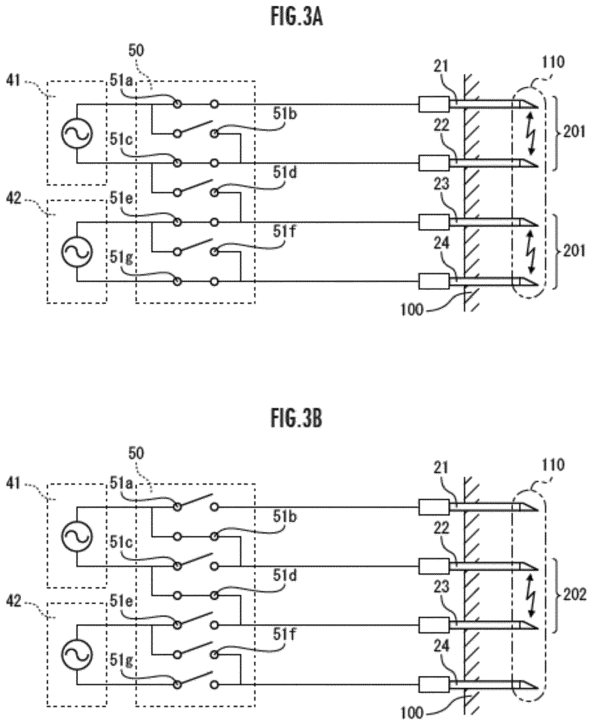

FIG. 3 is a schematic diagram illustrating operating states in quadripolar.

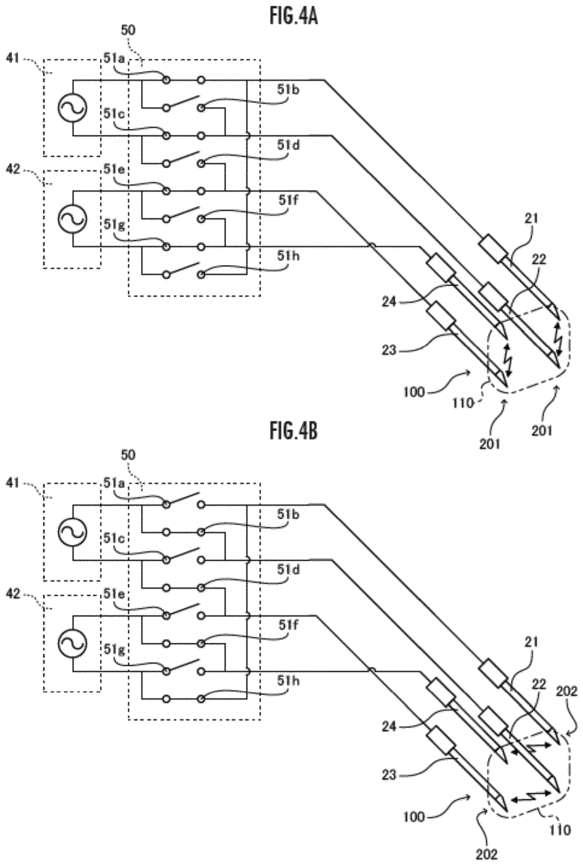

FIG. 4 is a schematic diagram illustrating a modification of the high-frequency treatment device.

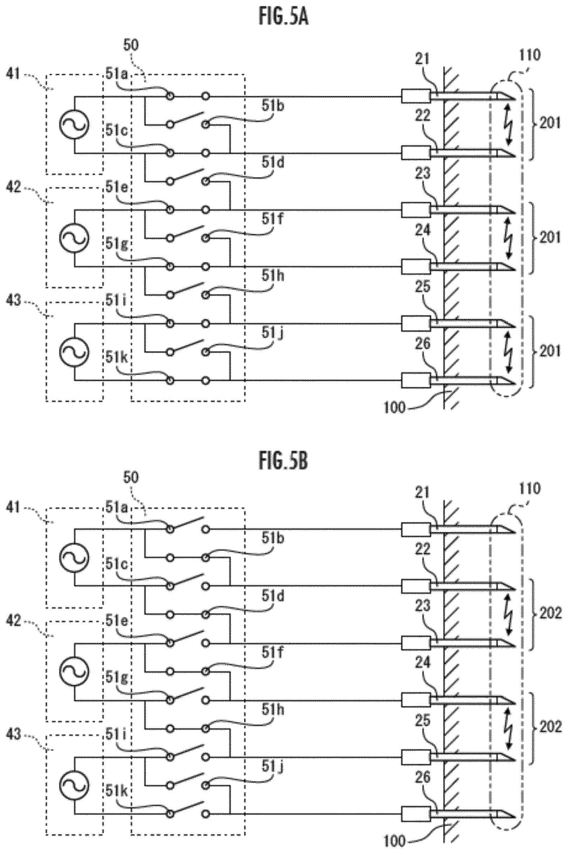

FIG. 5 is a schematic diagram illustrating another modification of the high-frequency treatment device.

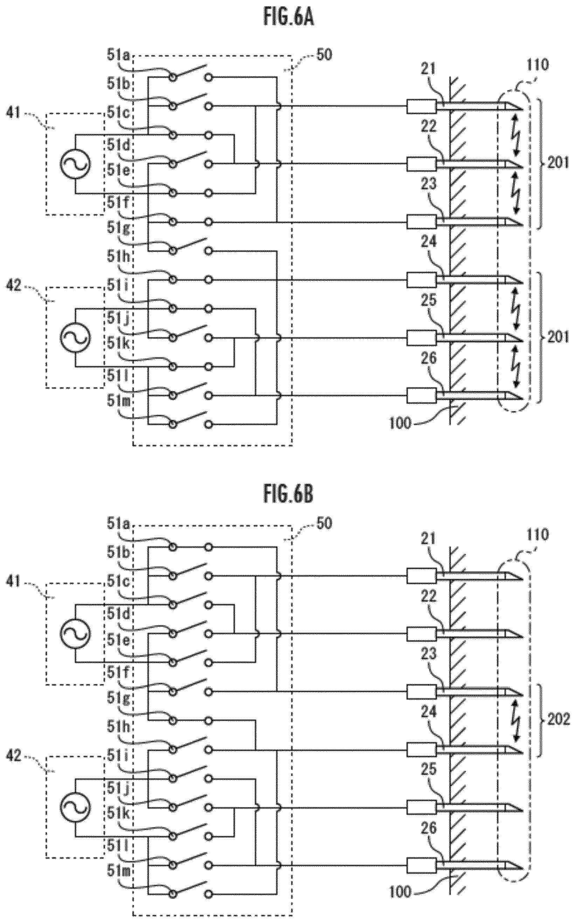

FIG. 6 is a schematic diagram illustrating still another modification of the high-frequency treatment device.

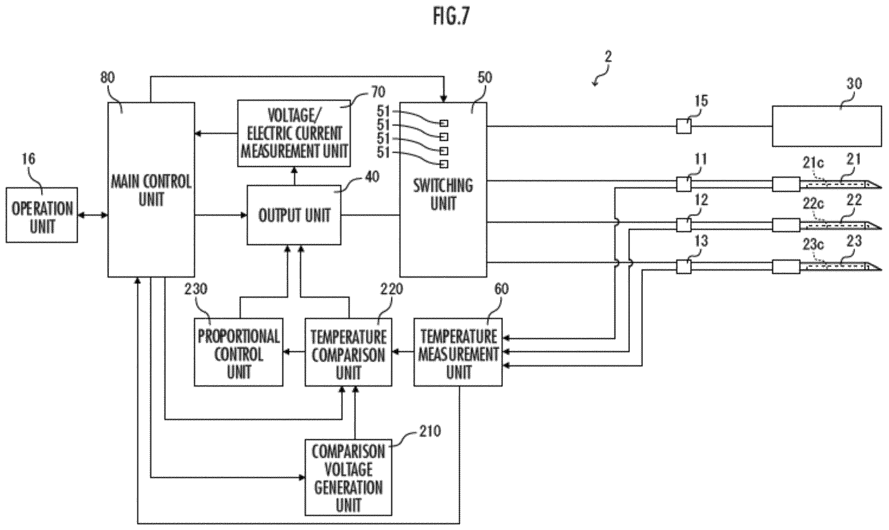

FIG. 7 is a block diagram illustrating the internal configuration of a high-frequency treatment device according to the present invention.

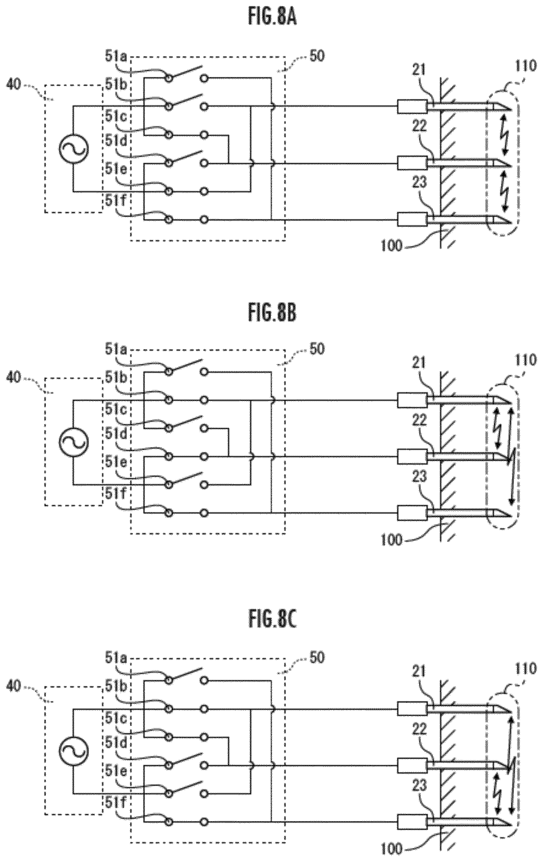

FIG. 8 is a schematic diagram illustrating operating states in tripolar.

### Description of Embodiments

The present disclosure will be described below with reference to the accompanying drawings.

FIG. 1 is a schematic diagram illustrating the appearance of a high-frequency treatment device 1. The high-frequency treatment device 1 partially heats peripheral nerves by a high-frequency electric current to perform a nerve block. As illustrated in FIG. 1, the high-frequency treatment device 1 includes a main body 10, four electrodes 21, 22, 23, and 24 connected to the main body 10, and a dispersive electrode 30 connected to the main body 10.

The main body 10 houses and supports internal components to be described later. Four electrode connectors 11 to 14 to which respective of the electrodes 21 to 24 are connected, and a dispersive electrode connector 15 connected to the dispersive electrode 30 are provided in a lower part of the front face of the main body 10. An operation unit 16 that accepts user operations is also provided on the front face of the main body 10. The operation unit 16 is composed of a touch panel display 16a that accepts input operations such as for various settings and displays various information, buttons 16b that accept operations to start and end of treatment, and a control nob 16c for adjusting output power.

A handle 17 used to carry the main body 10 is provided at the top of the main body 10. Further, though not illustrated, a power connector and a power switch are provided on the back face of the main body 10, which are connected to the commercial AC power supply to receive the supply of power.

The electrodes 21 to 24 are inserted into a human body, which is subjected to high-frequency treatment, to make a high-frequency electric current flow into the human body. The electrodes 21 to 24 are formed in a tubular needle shape to be able to inject drugs through the electrodes 21 to 24. Most sections of the electrodes 21 to 24 are insulating sections 21b to 24b coated with insulation except for non-insulating sections 21a to 24a at respective tips so that the high-frequency electric current is output from the non-insulating sections 21a to 24a.

Further, thermocouples 21c to 24c are incorporated in the electrodes 21 to 24 to measure treatment temperature. The electrodes 21 to 24 are electrically connected to the main body 10 through electrode cables 28 and the electrode connectors 11 to 14. Note that the electrodes 21 to 24 may also be formed in any other shape, for example, which may be formed into a rod shape inserted into a needle tube or a catheter. Further, the thermocouples 21c to 24c may be provided separately from the electrodes 21 to 24.

The dispersive electrode 30 is a flat-plate electrode pasted and placed on the skin surface of the human body to make the high-frequency electric current flow with the electrodes 21 to 24 inserted inside. In other words, the dispersive electrode 30 is used to make the high-frequency electric current flow in a so-called monopolar manner. The dispersive electrode 30 is electrically connected to the main body 10 through a dispersive electrode cable 31 and the dispersive electrode connector 15. Note that the dispersive electrode 30 is formed into a rectangular flat-plate shape, but the shape of the dispersive electrode 30 may be any other shape.

FIG. 2 is a block diagram illustrating the internal configuration of the high-frequency treatment device 1. As illustrated in FIG. 2, the high-frequency treatment device 1 includes, as internal components, a first output unit 41 and a second output unit 42, a switching unit 50, a first temperature measurement unit 61 and a second temperature measurement unit 62, a temperature reference signal generation unit 63, a temperature abnormality detection unit 64, a voltage measurement unit 71, an electric current measurement unit 72, a main control unit 80, and a sub-control unit 90.

Based on power supplied from the commercial AC power supply, the first output unit 41 and the second output unit 42 generate and output high-frequency power with a preset frequency (for example, 470 to 490 kHz) and a voltage (for example, 18 to 22 Vrms) based on an output control signal from the main control unit 80. Each of the first output unit 41 and the second output unit 42 is composed of a known circuit having a transformer, respectively, so that the human body to be treated is insulated from the commercial AC power supply.

The first output unit 41 is connected to the electrode connector 11, the electrode connector 12, the electrode connector 13, and the dispersive electrode connector 15 through the switching unit 50, that is, connected in such a manner to be able to output high-frequency power to the electrode 21, the electrode 22, the electrode 23, and the dispersive electrode 30. Further, the second output unit 42 is connected to the electrode connector 13, the electrode connector 14, and the dispersive electrode connector 15 through the switching unit 50, that is, connected in such a manner as to be able to output high-frequency power to the electrode 23, the electrode 24, and the dispersive electrode 30.

The first output unit 41 and the second output unit 42 constitute an output unit. Two output units, that is, the first output unit 41 and the second output unit 42, are provided to be able to make high-frequency electric current flow while performing output control on two electrode sets (for example, an electrode set of the electrode 21 and the electrode 22, and an electrode set of the electrode 23 and the electrode 24) stably at the same time.

The switching unit 50 operates under the control of the sub-control unit 90 to switch the connections of the first output unit 41 and the second output unit 42 to the electrode connectors 11 to 14 and the dispersive electrode connector 15. In other words, the switching unit 50 switches to which electrode set the high-frequency electric current is applied among electrode sets, in each of which at least two of the electrodes 21 to 24 and the dispersive electrode 30 are combined.

The switching unit 50 is composed of a circuit including multiple switches 51 each consisting of a semiconductor switch such as an MOSFET (Metal Oxide Semiconductor Field Effect Transistor) or a reed relay, and provided between the first output unit 41 and the second output unit 42, and the electrodes 21 to 24 and the dispersive electrode 30.

Since the two output units, that is, the first output unit 41 and the second output unit 42, are provided, the number of switches 51 can be reduced to simplify the structure of the switching unit 50 so as to speed up the switching of the connections of the first output unit 41 and the second output unit 42 to each of the electrodes 21 to 24.

Each of the first temperature measurement unit 61 and the second temperature measurement unit 62 is composed of a known circuit to generate a temperature measurement signal (for example, 25 mV/°C) based on the signals received from the thermocouples 21c to 24c, and transmit the temperature measurement signal to the sub-control unit 90 and the temperature abnormality detection unit 64, respectively. The first temperature measurement unit 61 is connected to the thermocouple 21c built in the electrode 21 and the thermocouple 23c built in the electrode 23. Further, the second temperature measurement unit 62 is connected to the thermocouple 22c built in the electrode 22 and the thermocouple 24c built in the electrode 24.

Two temperature measurement units, that is, the first temperature measurement unit 61 and the second temperature measurement unit 62, are provided to be able to measure temperature around all the four electrodes 21 to 24 accurately in a short time. In other words, the overall measurement time is prevented from becoming too long while ensuring a sufficient measurement time for each of the thermocouples 21c to 24c.

Further, since the first temperature measurement unit 61 and the second temperature measurement unit 62 measure both the temperature around the electrode 21, 22 or 23 to which high-frequency power is output from the first output unit 41, and the temperature around the electrode 23 or 24 to which high-frequency power is output from the second output unit 42, respectively, the occurrence of temperature abnormality by the first output unit 41 or the second output unit 42 can be detected even if either one of the first temperature measurement unit 61 and the second temperature measurement unit 62 is broken down, thus improving safety.

The temperature reference signal generation unit 63 is composed of a known circuit to generate a temperature reference signal as a reference for determining whether the temperature measured by the first temperature measurement unit 61 or the second temperature measurement unit 62 is an abnormal temperature or not. The temperature reference signal generation unit 63 is controlled by the sub-control unit 90 to generate a temperature reference signal corresponding, for example, to a temperature set value which is a control target for the main control unit 80 plus 7 °C. The generated temperature reference signal is transmitted to the temperature abnormality detection unit 64.

The temperature abnormality detection unit 64 is composed of a known circuit to compare the temperature reference signal received from the temperature reference signal generation unit 63 with the temperature measurement signal received from the first temperature measurement unit 61 or the second temperature measurement unit 62 so as to detect the occurrence of temperature abnormality. When the voltage of the temperature measurement signal is higher than the voltage of the temperature reference signal, the temperature abnormality detection unit 64 transmits an output stop signal to the first output unit 41 and the second output unit 42, and the main control unit 80. The first output unit 41 and the second output unit 42 that received the output stop signal stop outputting the high-frequency power. Further, the main control unit 80 that received the output stop signal performs temperature abnormality processing such as alarm indication.

The voltage measurement unit 71 is composed of a known circuit to individually measure a voltage of high-frequency power output from each of the first output unit 41 and the second output unit 42. The voltage measurement unit 71 generates a voltage measurement signal based on the high-frequency voltages generated by the first output unit 41 and the second output unit 42, and transmits the voltage measurement signal to the main control unit 80.

The electric current measurement unit 72 is composed of a known circuit to individually measure a high-frequency electric current flowing into each of the electrodes 21 to 24. The electric current measurement unit 72 generates an electric current measurement signal based on the high-frequency electric currents flowing into the electrodes 21 to 24, and transmits the electric current measurement signal to the main control unit 80.

The main control unit 80 includes known components such as a CPU, a ROM, and a RAM to control each of units of the high-frequency treatment device 1, such as the operation unit 16, the first output unit 41, and the second output unit 42 in order to execute high-frequency treatment. Based on an input operation accepted by the operation unit 16, the main control unit 80 transmits, to the sub-control unit 90, a connection mode signal indicative of each of the connection modes of the first output unit 41 and the second output unit 42 to the electrode connectors 11 to 14 and the dispersive electrode connector 15.

Further, based on an input operation accepted by the operation unit 16, the main control unit 80 starts or finishes outputting high-frequency power from the first output unit 41 or the second output unit 42, and performs output control of the high-frequency power by PID control based on the temperature set value input to the operation unit 16 and the temperature measured value received from the sub-control unit 90 to make the temperature measured value approximately equal to the temperature set value. Note that the main control unit 80 sets the output voltage as a manipulated variable in the radiofrequency thermocoagulation and sets the output pulse width as a manipulated variable in the pulsed radiofrequency.

Further, based on the voltage measurement signal received from the voltage measurement unit 71 and the electric current measurement signal received from the electric current measurement unit 72, the main control unit 80 calculates a voltage measured value, an electric current measured value, a power measured value, and an impedance measured value, and displays the calculated values on the touch panel display 16a together with the temperature measured value. Note that, when an output voltage value is set with an input operation accepted by the operation unit 16, the main control unit 80 performs output control of the high-frequency power by PID control to make the voltage measured value equal to the voltage set value.

Like the main control unit 80, the sub-control unit 90 includes known components such as a CPU, a ROM, and a RAM to control the switching unit 50, the first temperature measurement unit 61, the second temperature measurement unit 62, and the temperature reference signal generation unit 63. Based on the connection mode signal transmitted from the main control unit 80, the sub-control unit 90 controls on/off of the multiple switches 51 included in the switching unit 50 to switch the connection state of the first output unit 41 and the second output unit 42 to the electrode connectors 11 to 14 and the dispersive electrode connector 15. In other words, the sub-control unit 90 constitutes part of the control unit.

Further, based on the temperature measurement signal received from the first temperature measurement unit 61 or the second temperature measurement unit 62, the sub-control unit 90 calculates and transmits, to the main control unit 80, a temperature measured value. Further, based on the temperature set value received from the main control unit 80, the sub-control unit 90 controls the temperature reference signal generation unit 63 to generate and transmit, to the temperature abnormality detection unit 64, a temperature reference signal.

Note that the switching unit 50, the first temperature measurement unit 61, the second temperature measurement unit 62, the temperature reference signal generation unit 63, the temperature abnormality detection unit 64, the voltage measurement unit 71, the electric current measurement unit 72, and the sub-control unit 90 are insulated from the commercial AC power supply to protect the human body to be treated.

Next, the operation of the high-frequency treatment device 1 will be described.

The high-frequency treatment device 1 can use four kinds of output formats of high-frequency electric current to an object to be treated: monopolar, bipolar, tripolar, and quadripolar. The monopolar is an output format to make the high-frequency electric current flow into an electrode set in which any one of the electrodes 21 to 24 is combined with the dispersive electrode 30, and the bipolar is an output format to make the high-frequency electric current flow into the electrode 21 and the electrode 22, or the electrode 23 and the electrode 24 as an electrode set. Further, the tripolar is an output format to set the electrode 21, the electrode 22, and the electrode 23 as an electrode set so as to make the high-frequency electric current flow between the electrode 21 and the electrodes 22, 23, between the electrode 22 and the electrodes 21, 23 or between the electrode 23 and the electrodes 21, 22.

The operation in the quadripolar will be described below. FIG. 3A and FIG. 3B are schematic diagrams illustrating operating states in the quadripolar. Note that only switches 51a to 51g necessary for description among the multiple switches 51 included in the switching unit 50 are illustrated in FIG. 3A and FIG. 3B for ease of understanding.

As illustrated in these figures, when high-frequency treatment is performed by the quadripolar, all the four electrodes 21 to 24 are first inserted into an object 100 to be treated such as the human body. At this time, the electrodes 21 to 24 are arranged in line at substantially equal intervals in this order. Although nerve exploration, and the measurement of impedance of surrounding tissue are performed when arranging the electrodes 21 to 24, the description thereof will be omitted because they are conventional technology.

When the electrodes 21 to 24 are arranged properly and the operation unit 16 accepts a quadripolar selection operation from a user such as a doctor, the main control unit 80 transmits, to the sub-control unit 90, a connection mode signal indicative of a quadripolar connection mode. When receiving the connection mode signal indicative of the quadripolar connection mode from the main control unit 80, the sub-control unit 90 controls the switching unit 50 to set the connection state of the first output unit 41 and the second output unit 42 to the electrodes 21 to 24 to a first state illustrated in FIG. 3A.

Specifically, in the first state, the sub-control unit 90 turns on the switch 51a, the switch 51c, the switch 51e, and the switch 51g, and turns off the switch 51b, the switch 51d, and the switch 51f. Thus, the first output unit 41 is connected to the electrode 21 and the electrode 22, and the second output unit 42 is connected to the electrode 23 and the electrode 24. Therefore, the high-frequency electric current flows between the electrode 21 and the electrode 22 by the high-frequency power output from the first output unit 41, and the high-frequency electric current flows between the electrode 23 and the electrode 24 by the high-frequency power output from the second output unit 42. As a result, the electrode 21 and the electrode 22, and the electrode 23 and the electrode 24 each make up a first electrode set 201 as a set of electrodes between which the high-frequency electric current flows in the first state, respectively.

Then, after the operation unit 16 accepts a selection operation of the radiofrequency thermocoagulation or the pulsed radiofrequency and input operations of the temperature set value (for example, 80 °C) and the treatment time (for example, 3 minutes) by the user (in the case where the pulsed radiofrequency is selected, only the treatment time is input because the main control unit 80 sets the temperature set value to 42 °C), when the operation unit 16 accepts a treatment start operation, the main control unit 80 controls the first output unit 41 and the second output unit 42 to start outputting the high-frequency power. Further, the main control unit 80 transmits, to the sub-control unit 90, an output start signal indicating that output is started.

When receiving the output start signal from the main control unit 80, the sub-control unit 90 controls the switching unit 50 to switch the connection state of the first output unit 41 and the second output unit 42 to the electrodes 21 to 24 between the first state illustrated in FIG. 3A and a second state illustrated in FIG. 3B in a specific cycle (0.1 seconds).

As illustrated in FIG. 3B, in the second state, the sub-control unit 90 turns on the switch 51b and the switch 51d, and turns off the switch 51a, the switch 51c, the switch 51e, the switch 51f, and the switch 51g. Thus, the first output unit 41 is connected to the electrode 22 and the electrode 23, and the high-frequency electric current flows between the electrode 22 and the electrode 23 by the high-frequency power output from the first output unit 41. As a result, the electrode 22 and the electrode 23 make up a second electrode set 202 as a set of electrodes between which the high-frequency electric current flows in the second state.

During outputting the high-frequency power, the first state in which the high-frequency electric current flows between the electrode 21 and the electrode 22, and between the electrode 23 and the electrode 24, and the second state in which the high-frequency electric current flows between the electrode 22 and the electrode 23 are switched by switching control of the switching unit 50 by the sub-control unit 90. Thus, although heat is intermittently applied to an area between and around the electrode 21 and the electrode 22, an area between and around the electrode 22 and the electrode 23, and an area between and around the electrode 23 and the electrode 24, respectively, the switching cycle is set to 0.1 seconds to prevent the temperature in each area from dropping too low while being not heated.

After the temperature measured value measured by each of the thermocouples 21c to 24c respectively built in the electrodes 21 to 24 rises to a temperature approximately equal to the temperature set value, the main control unit 80 controls the output of the first output unit 41 and the output of the second output unit 42 to maintain such a state that the temperature measured value is approximately equal to the temperature set value. Thus, while the high-frequency power is being output, an area 110 between and around the electrodes 21 to 24 is maintained at a temperature approximately equal to the temperature set value as a whole so as to cause thermocoagulation or degeneration of tissue.

When the treatment time input beforehand has passed after the start of output of the high-frequency power, the main control unit 80 stops outputting the high-frequency power from the first output unit 41 and the second output unit 42. Further, the main control unit 80 transmits an output end signal to the sub-control unit 90. The sub-control unit 90 that received the output end signal from the main control unit 80 stops the switching control of the switching unit 50 between the first state and the second state. One treatment is completed as above. Nerve tissue included in the area 110 becomes such a state that a pain signal is blocked as a result of being heated for 3 minutes at a temperature of 80 °C, for example.

Thus, according to the high-frequency treatment by the quadripolar, heat can be applied to the wide area 110 between and around the electrodes 21 to 24 with one treatment. For example, when the area 110 is heated with high-frequency treatment by the bipolar, a total of three treatments, that is, treatment to make the high-frequency electric current flow between the electrode 21 and the electrode 22, treatment to make the high-frequency electric flow between the electrode 22 and the electrode 23, and treatment to make the high-frequency electric current flow between the electrode 23 and the electrode 24, are required. On the other hand, in the high-frequency treatment by the quadripolar, since only one treatment is required, the time required to the treatment can be reduced to about 1/3.

Therefore, according to the high-frequency treatment device 1, heating can be performed extremely efficiently even in such as a nerve block for sacroiliac joint pain, which requires to heat a wide area, and this also reduces the physical burden on the human body as the object to be treated.

Further, since the electrodes 21 to 24 are arranged in order in such a manner that the electrode 22 and the electrode 23 that make up the second electrode set 202 are placed next to each other, overlapping between the heated area in the first state and the heated area in the second state is minimized to enable a wider area 110 to be heated.

Further, since only the electrodes 21 to 24 inserted into the object 100 to be treated are used to apply heat in the same manner as the bipolar in both the first state and the second state, the area 110 wider than that when the dispersive electrode 30 is used can be heated.

Further, since an output unit is provided for each of first electrode sets 201 by the first output unit 41 and the second output unit 42, respectively, the high-frequency electric current can be made to flow while stably performing output control in the two first electrode sets 201 at the same time without being affected by an impedance difference between the two first electrode sets 201.

The switching cycle is preferred to be 0.3 seconds or less, more preferred to be 0.2 seconds or less, and most preferred to be 0.1 seconds or less from the viewpoint of suppressing the temperature drop in an unheated area. Note further that the lower limit of the switching cycle can be set according to the operating speed of the switching unit 50, the frequency of the high-frequency power to be output, or the pulse width in the pulsed radiofrequency.

Next, modifications of the high-frequency treatment device 1 will be described.

FIG. 4A and FIG. 4B, FIG. 5A and FIG. 5B, and FIG. 6A and FIG. 6B are schematic diagrams illustrating the modifications of the high-frequency treatment device 1. Note that only switches 51a to 51h are illustrated in FIG. 4A and FIG. 4B, only switches 51a to 51k are illustrated in FIG. 5A and FIG. 5B, and only switches 51a to 51m are illustrated in FIG. 6A and FIG. 6B for ease of understanding.

In the example illustrated in FIG. 4A and FIG. 4B, the electrode 21 and the electrode 22, and the electrode 23 and the electrode 24 make up the first electrode sets 201 in the first state, and the electrode 21 and the electrode 24, and the electrode 22 and the electrode 23 make up the second electrode sets 202 in the second state, respectively.

Specifically, in this example, the sub-control unit 90 turns on the switch 51a, the switch 51c, the switch 51e, and the switch 51g, and turns off the switch 51b, the switch 51d, the switch 51f, and the switch 51h in the first state as illustrated in FIG. 4A.

Thus, in the first state, the first output unit 41 is connected to the electrode 21 and the electrode 22, and the second output unit 42 is connected to the electrode 23 and the electrode 24, so that the high-frequency electric current flows between the electrode 21 and the electrode 22 by the high-frequency power output from the first output unit 41, and the high-frequency electric current flows between the electrode 23 and the electrode 24 by the high-frequency power output from the second output unit 42.

Further, in this example, the sub-control unit 90 turns on the switch 51b, the switch 51d, the switch 51f, and the switch 51h, and turns off the switch 51a, the switch 51c, the switch 51e, and the switch 51g in the second state as illustrated in FIG. 4B.

Thus, in the second state, the first output unit 41 is connected to the electrode 22 and the electrode 23, and the second output unit 42 is connected to the electrode 24 and the electrode 21, so that the high-frequency electric current flows between the electrode 22 and the electrode 23 by the high-frequency power output from the first output unit 41, and the high-frequency electric current flows between the electrode 24 and the electrode 21 by the high-frequency power output from the second output unit 42.

Thus, two second electrode sets may also be generated in the second state. In this case, the electrodes 21 to 24 are arranged in two lines as illustrated in FIG. 4A and FIG. 4B to enable a rectangular area 110 to be heated.

In the example illustrated in FIG. 5A and FIG. 5B, a third output unit 43 is added and six electrodes 21 to 26 are used. Then, the electrode 21 and the electrode 22, the electrode 23 and the electrode 24, and the electrode 25 and the electrode 26 make up first electrode sets 201 in the first state, and the electrode 22 and the electrode 23, and the electrode 24 and the electrode 25 make up second electrode sets 202 in the second state, respectively.

Specifically, in this example, the sub-control unit 90 turns on the switch 51a, the switch 51c, the switch 51e, the switch 51g, the switch 51i, and the switch 51k, and turns off the switch 51b, the switch 51d, the switch 51f, the switch 51h, and the switch 51j in the first state as illustrated in FIG. 5A.

Thus, in the first state, the first output unit 41 is connected to the electrode 21 and the electrode 22, the second output unit 42 is connected to the electrode 23 and the electrode 24, and the third output unit 43 is connected to the electrode 25 and the electrode 26. As a result, the high-frequency electric current flows between the electrode 21 and the electrode 22 by the high-frequency power output from the first output unit 41, the high-frequency electric current flows between the electrode 23 and the electrode 24 by the high-frequency power output from the second output unit 42, and the high-frequency electric current flows between the electrode 25 and the electrode 26 by the high-frequency power output from the third output unit 43.

Further, in this example, the sub-control unit 90 turns on the switch 51b, the switch 51d, the switch 51f, and the switch 51h, and turns off the switch 51a, the switch 51c, the switch 51e, the switch 51g, the switch 51i, the switch 51j, and the switch 51k in the second state as illustrated in FIG. 5B.

Thus, in the second state, the first output unit 41 is connected to the electrode 22 and the electrode 23, and the second output unit 42 is connected to the electrode 24 and the electrode 25, so that the high-frequency electric current flows between the electrode 22 and the electrode 23 by the high-frequency power output from the first output unit 41, and the high-frequency electric current flows between the electrode 24 and the electrode 25 by the high-frequency power output from the second output unit 42.

Thus, more electrodes 21 to 26 are used to increase the numbers of first electrode sets 201 and second electrode sets 202 to enable a wider area 110 to be heated with one treatment. Further, each of the output units (the first output unit 41, the second output unit 42, and the third output unit 43) is provided for each of three first electrode sets 201 even in this case to enable the high-frequency electric current to flow while stably performing output control in the three first electrode sets 201 at the same time. Further, the structure of the switching unit 50 can be simplified to speed up switching.

The number of electrodes is required to be at least four or more because there is a need to generate two first electrode sets 201 or more in the first state in order to generate at least one second electrode set 202 in the second state.

In the example illustrated in FIG. 6A and FIG. 6B, six electrodes 21 to 26 are so used that a set of the electrode 21, the electrode 22, and the electrode 23, and a set of the electrode 24, the electrode 25, and the electrode 26 make up first electrode sets 201 in the first state, respectively, and the electrode 23 and the electrode 24 make up a second electrode set 202 in the second state.

Specifically, in this example, the sub-control unit 90 turns on the switch 51c, the switch 51e, the switch 51f, the switch 51h, the switch 51i, and the switch 51k, and turns off the switch 51a, the switch 51b, the switch 51d, the switch 51g, the switch 51j, the switch 511, and the switch 51m in the first state as illustrated in FIG. 6A.

Thus, in the first state, the first output unit 41 is connected to the electrode 21, the electrode 22, and the electrode 23, and the second output unit 42 is connected to the electrode 24, the electrode 25, and the electrode 26. As a result, the high-frequency electric current flows between the electrode 22 and the electrodes 21, 23 by the high-frequency power output from the first output unit 41, and the high-frequency electric current flows between the electrode 25 and the electrodes 24, 26 by the high-frequency power output from the second output unit 42.

Further, in this example, the sub-control unit 90 turns on the switch 51a and the switch 51g, and turns off the switches 51b to 51f, and the switches 51h to 51m in the second state as illustrated in FIG. 6B. Thus, in the second state, the first output unit 41 is connected to the electrode 23 and the electrode 24, and the high-frequency electric current flows between the electrode 23 and the electrode 24 by the high-frequency power output from the first output unit 41.

Thus, each first electrode set 201 may also be made up of three electrodes, and even in this case, a wide area 110 equivalent to that in the example illustrated in FIG. 5A and FIG. 5B can be heated. Further, in this case, since output control can be performed stably by two output units, that is, the first output unit 41 and the second output unit 42, the cost can be reduced even more than that in the example illustrated in FIG. 5A and FIG. 5B.

In this example, for example, a set of the electrode 21, the electrode 22, and the electrode 23, and a set of the electrode 25 and the electrode 26 may be set as first electrode sets 201, and a set of the electrode 23, the electrode 24, and the electrode 25 may be set as a second electrode set 202. In other words, the second electrode set 202 may also be made up of three electrodes.

Alternatively, though not illustrated, the electrodes 21 to 23 and the dispersive electrode 30 may be used, then a set of the electrode 21 and the electrode 22, and a set of the electrode 23 and the dispersive electrode 30 may be set as first electrode sets 201, and the electrode 22 and the electrode 23, or the electrode 22 and the dispersive electrode 30 may be set as a second electrode set 202. In other words, each of the first electrode sets 201 and the second electrode set 202 may also include the dispersive electrode 30.

Father, the switching unit 50 may be structured as appropriate, and , for example, in the arrangement of the electrodes 21 to 24 illustrated in FIG. 3A and FIG. 3B, a set of the electrode 21 and the electrode 23, and a set of the electrode 22 and the electrode 24 may be set as first electrode sets 201, and the electrode 21 and the electrode 24 may be set as a second electrode set 202 Similarly, for example, in the arrangement of the electrodes 21 to 24 illustrated in FIG. 4A and FIG. 4B, a set of the electrode 21 and the electrode 22, and a set of the electrode 23 and the electrode 24 may be set as first electrode sets 201, and a set of the electrode 21 and the electrode 23, and a set of the electrode 22 and the electrode 24 may be set as second electrode sets 202.

Further, the electrodes 21 to 26 may also be placed, for example, on the skin surface of a human body to heat a relatively shallow area under the skin.

Next, the high-frequency treatment device 2 of the present invention which is defined in appended claim 1 will be described.

Like the high-frequency treatment device 1, a high-frequency treatment device 2 according to the present invention is to partially heat peripheral nerves using a high-frequency electric current to perform a nerve block. Since the high-frequency treatment device 2 basically has the same structure as the high-frequency treatment device 1, except that only three electrodes 21, 22, and 23 are included, and that the internal configuration is different, parts identical to those above are given the same reference numerals to omit the description thereof. In the following, only parts different from those abovewill be described.

FIG. 7 is a block diagram illustrating the internal configuration of the high-frequency treatment device 2. As illustrated in this figure, the high-frequency treatment device 2 includes, as internal components, an output unit 40, the switching unit 50, a temperature measurement unit 60, a comparison voltage generation unit 210, a temperature comparison unit 220, a proportional control unit 230, a voltage/electric current measurement unit 70, and the main control unit 80.

The output unit 40 has a structure similar to the first output unit 41 and the second output unit 42 to generate and output, based on power supplied from the commercial AC power supply, high-frequency power with a preset frequency (for example, 470 to 490 kHz) and a voltage (for example, 18 to 22 Vrms) based on an output control signal form the main control unit 80 and a pulse signal from the proportional control unit 230. The output unit 40 is connected to electrode connectors 11 to 13 and the dispersive electrode connector 15 through the switching unit 50, that is, connected in a manner to be able to output high-frequency power to electrodes 21 to 23 and the dispersive electrode 30.

The switching unit 50 is composed of a circuit including multiple switches 51 each consisting of a semiconductor switch such as an MOSFET or a reed relay, and provided between the output unit 40, and the electrodes 21 to 23 and the dispersive electrode 30. In the present embodiment, the switching unit 50 operates under the control of the main control unit 80 to switch the connection of the output unit 40 to the electrode connectors 11 to 13 and the dispersive electrode connector 15.

The temperature measurement unit 60 is composed of a known circuit to generate and transmit, to the main control unit 80, a temperature measurement signal of, for example, 40 mV/°C based on signals received from thermocouples 21c to 23c, and to generate and transmit, to the temperature comparison unit 220, a temperature measurement signal of, for example, 20 mV/°C.

The comparison voltage generation unit 210 is composed of a known circuit to generate a control reference voltage signal used for output control of the output unit 40, and a determination reference voltage signal used for abnormality determination on measured temperature. The comparison voltage generation unit 210 is controlled by the main control unit 80 to generate and transmit, to the temperature comparison unit 220, a control reference voltage signal (voltage indicative of a temperature set value) and a voltage signal used for determination (for example, voltage indicative of a temperature set value plus 7 °C) based on the temperature set value received from the main control unit 80.

The temperature comparison unit 220 is composed of a known circuit to transmit, to the proportional control unit 230, a differential voltage signal indicative of a voltage difference between the control reference voltage signal received from the comparison voltage generation unit 210 and the temperature measurement signal received from the temperature measurement unit 60. The temperature comparison unit 220 also compares the voltage of the determination reference voltage signal received from the comparison voltage generation unit 210 with the voltage of the temperature measurement signal received from the temperature measurement unit 60, and transmits an output stop signal to the output unit 40 and the main control unit 80 when the voltage of the temperature measurement signal is higher. The output unit 40 that received the output stop signal stops outputting high-frequency power, and the main control unit 80 that received the output stop signal performs temperature abnormality processing such as alarm indication.

The proportional control unit 230 is composed of such as a known PWM (Pulse Width Modulation) circuit including a sawtooth-wave generator and a comparator to generate a pulse signal obtained by modulating a pulse width based on the differential voltage signal. The proportional control unit 230 compares the differential voltage signal received from the temperature comparison unit 220 with a sawtooth-wave signal to generate and transmit the pulse signal to the output unit 40.

The voltage/electric current measurement unit 70 is composed of a known circuit to measure the voltage and electric current of high-frequency power output from the output unit 40. Based on the high-frequency voltage and the high-frequency electric current generated by the output unit 40, the voltage/electric current measurement unit 70 generates and transmits, to the main control unit 80, a voltage measurement signal and an electric current measurement signal.

The main control unit 80 includes known components such as a CPU, a ROM, and a RAM to control each unit of the high-frequency treatment device 2 in order to execute high-frequency treatment. The main control unit 80 generates and transmits, to the output unit 40, an output control signal indicative of an output voltage according to preset output power or output power input to the operation unit 16 to perform output control of high-frequency power together with the proportional control unit 230. The output unit 40 generates a temperature control signal by synthesizing the pulse signal received from the proportional control unit 230 and the output control signal received from the main control unit 80, and smoothing the synthesized signal, and outputs high-frequency power of a voltage based on this temperature control signal. Further, based on the received voltage measurement signal and electric current measurement signal, the main control unit 80 calculates a voltage measured value, an electric current measured value, a power measured value, and an impedance measured value.

Further, in the present embodiment, the main control unit 80 constitutes the control unit of the present invention to perform control of the switching unit 50. Specifically, the main control unit 80 controls on/off of the multiple switches 51 included in the switching unit 50 based on an input operation accepted by the operation unit 16 to switch the connection state of the output unit 40 to the electrode connectors 11 to 13 and the dispersive electrode connector 15.

Next, the operation of the high-frequency treatment device 2 will be described.

The high-frequency treatment device 2 can use three kinds of output formats of high-frequency electric current to an object to be treated: monopolar, bipolar, and tripolar. The monopolar is an output format to make the high-frequency electric current flow into an electrode set in which any one of the electrodes 21 to 23 is combined with the dispersive electrode 30, and the bipolar is an output format to make the high-frequency electric current flow into the electrode 21 and the electrode 22 as an electrode set.

The operation of the tripolar as the feature of the present embodiment will be described below. FIG. 8A to FIG. 8C are schematic diagrams illustrating operating states in the tripolar. Note that only switches 51a to 51f necessary for description are illustrated in FIG. 8A to FIG. 8C for ease of understanding.

When the electrodes 21 to 23 are arranged properly and the operation unit 16 accepts a tripolar selection operation from a user such as a doctor, the main control unit 80 controls the switching unit 50 to put the connection state of the output unit 40 to the electrodes 21 to 23 into a state illustrated in FIG. 8A. Specifically, the main control unit 80 turns on the switch 51c, the switch 51e, and the switch 51f, and turns off the switch 51a, the switch 51b, and the switch 51d. Thus, the connection state is put into a state in which the high-frequency electric current flows between the electrode 22 and the electrodes 21, 23 in the electrode set consisting of the three electrodes 21 to 23 by the high-frequency power output from the output unit 40.

Then, after the operation unit 16 accepts a selection operation of the radiofrequency thermocoagulation or the pulsed radiofrequency by the user, and input operations of the temperature set value (for example, 80 °C) and the treatment time (for example, 3 minutes) (in the case where the pulsed radiofrequency is selected, only the treatment time is input because the main control unit 80 sets the temperature set value to 42 °C), when the operation unit 16 accepts a treatment start operation, the main control unit 80 controls the output unit 40 to start outputting high-frequency power.

Further, the main control unit 80 controls the switching unit 50 to switch the connection state of the output unit 40 to the electrodes 21 to 23 among three states illustrated in FIG. 8A to FIG. 8C in a specific cycle (0. 3 seconds in the present embodiment). Specifically, the state illustrated in FIG. 8B is a state in which the switch 51b, the switch 51d, and the switch 51f are turned on, and the switch 51a, the switch 51c, and the switch 51e are turned off to make the high-frequency electric current flow between the electrode 21 and the electrodes 22, 23. Further, the state illustrated in FIG. 8C is a state in which the switch 51b, the switch 51c, and the switch 51f are turned on, and the switch 51a, the switch 51d, and the switch 51e are turned off to make the high-frequency electric current flow between the electrode 23 and the electrodes 21, 22.

In other words, the main control unit 80 controls the switching unit 50 in such a manner that the high-frequency electric current flows between any one of the electrodes 21 to 23, which is switched in order in the specific cycle, and the remaining two electrodes during the output of the high-frequency power. Thus, heat is applied to an area between and around the electrode 21 and the electrode 22, an area between and around the electrode 22 and the electrode 23, and an area between and around the electrode 23 and the electrode 21.

After the temperature measured value measured by each of the thermocouples 21c to 23c built in each of the electrodes 21 to 23 rises to a temperature approximately equal to the temperature set value, the main control unit 80 controls the output of the output unit 40 together with the proportional control unit 230 to maintain such a state that the temperature measured value is approximately equal to the temperature set value.

In the present embodiment, the output control by the main control unit 80 and the proportional control unit 230 is performed based on the temperature measured value of the thermocouple 22c built in the electrode 22 in the state illustrated in FIG. 8A, the temperature measured value of the thermocouple 21c built in the electrode 21 in the state illustrated in FIG. 8B, and the temperature measured value of the thermocouple 23c built in the electrode 23 in the state illustrated in FIG. 8C, respectively. In other words, in the present embodiment, temperature around an electrode with the highest in electric current density among the electrodes 21 to 23 is referred to increase the accuracy and stability of the output control.

When the treatment time input beforehand has passed after the start of the output of the high-frequency power, the main control unit 80 stops the output of the high-frequency power from the output unit 40, and stops the switching control to the switching unit 50. One treatment is completed as above.

Thus, according to the high-frequency treatment by the tripolar, heat can be applied to the wide area 110 between and around the electrodes 21 to 23 with one treatment, which requires two treatments by the bipolar.

According to the high-frequency treatment by the tripolar, since heat can be applied between the electrode 21 and the electrode 22, between the electrode 22 and the electrode 23, and between the electrode 23 and the electrode 21 all with one treatment, it becomes possible that by arranging the electrodes 21 to 23 in, for example, a V shape, a triangular area 110 be heated.

Since the electrodes 21 to 23 (thermocouples 21c to 23c) to perform temperature measurement are switched in each of the three states in the present embodiment, it is preferred to shorten the temperature unmeasured time in order to monitor temperature properly. From this point of view, the switching cycle is preferred to be 0.5 seconds or less, more preferred to be 0.4 seconds or less, and most preferred to be 0.3 seconds or less. Note that the lower limit of the switching cycle can be set according to the operating speed of the switching unit 50, the frequency of the high-frequency power to be output, or the pulse width in the pulsed radiofrequency.

Further, the example of starting the treatment from the state illustrated in FIG. 8A is illustrated in the present embodiment, but it is needless to say that the treatment may be started from the state illustrated in FIG. 8B or FIG. 8C. Further, any of the states illustrated in FIG. 8A to FIG. 8C may be maintained without switching among the three states during the treatment.

Further, the example of the high-frequency treatment device 2 including the three electrodes 21 to 23 is illustrated in the present embodiment, but the number of electrodes included in the high-frequency treatment device 2 may be four or more. In this case, for example, any three of four electrodes may make up an electrode set to make the high-frequency electric current flow between one electrode and remaining two electrodes included in this electrode set, or all the four electrodes may make up an electrode set to make the high-frequency electric current flow between one electrode and remaining three electrodes included in this electrode set.

For example, with respect to the shapes and arrangements of respective elements in the high-frequency treatment device 2 various known shapes and arrangements can be adopted. Further, the high-frequency treatment device 2 may also be used for any other purpose such as tumor ablation.

Further, the high-frequency treatment device 2 may include two output units 40 and two temperature measurement units 60, and may include the sub-control unit 90 for controlling the switching unit 50, and the temperature measurement unit 60.

### Description of Reference Numerals

- 1, 2: high-frequency treatment device
- 21 to 26: electrode
- 30: dispersive electrode
- 40: output unit
- 41: first output unit
- 42: second output unit
- 50: switching unit
- 80: main control unit
- 90: sub-control unit
- 100: object to be treated
- 201: first electrode set
- 202: second electrode set

## Claims

1. A high-frequency treatment device (2) comprising:
an output unit (40) which is configured to output high-frequency power;
at least three electrodes (21, 22, 23) connected to the output unit (40) and arranged in an object to be treated (100);
a switching unit (50) provided between the output unit (40) and the electrodes (21, 22, 23) to switch into which electrode set among electrode sets with at least three of the electrodes (21, 22, 23) combined a high-frequency electric current flows; and
a control unit (80) which is configured to control the switching unit (50) to make the high-frequency electric current flow between any specific one of the electrodes (21, 22, 23) included in a specific first electrode set (201), in which at least three of the electrodes (21, 22, 23) are combined, and all the remaining electrodes included in the specific electrode set,
**characterized in that**
the control unit (80) is configured to control the switching unit (50) to switch, in a specific cycle during treatment, between first states in which the high-frequency electric current first flows into one of at least two first electrode sets (201) and then into another of the at least two first electrode sets (201), and a second state in which the high-frequency electric current flows into a second electrode set (202) including the specific one of the electrodes (21, 22, 23) included in one of the first electrode sets (201) and the specific one of the electrodes (21, 22, 23) included in the other one of the first electrode sets (201).

2. The high-frequency treatment device (2) according to claim 1, wherein the second electrode set (202) includes electrodes placed next to each other.

3. The high-frequency treatment device according to claim 1 or 2, wherein the electrodes (21, 22, 23) are inserted into the object to be treated (100).

4. The high-frequency treatment device (2) according to any one of claims 1 to 3, wherein the control unit (80) switches between the first state and the second state in a cycle of 0.3 seconds or less.

5. The high-frequency treatment device (2) according to any one of claims 1 to 4, wherein the output unit (40) is provided for each of the first electrode sets (201).

## Patentansprüche

1. Vorrichtung zur Hochfrequenzbehandlung (2), umfassend:
eine Ausgabeeinheit (40), die konfiguriert ist, Hochfrequenzenergie auszugeben,
mindestens drei Elektroden (21, 22, 23), die mit der Ausgabeeinheit (40) verbunden sind und in einem zu behandelnden Subjekt (100) angeordnet sind,
eine Schalteinheit (50), die zwischen der Ausgabeeinheit (40) und den Elektroden (21, 22, 23) vorgesehen ist, um eine Schaltung durchzuführen, in welchen Elektrodensatz unter den Elektrodensätzen, die mindestens drei der Elektroden (21, 22, 23) in Kombination aufweisen, ein elektrischer Hochfrequenzstrom fließt, und
eine Steuereinheit (80), die konfiguriert ist, die Schalteinheit (50) zu steuern, um den elektrischen Hochfrequenzstrom zwischen einer beliebigen speziellen (Elektrode) der Elektroden (21, 22, 23), die in einem speziellen ersten Elektrodensatz (201), in dem mindestens drei der Elektroden (21, 22, 23) kombiniert sind, enthalten ist, und allen übrigen Elektroden, die in dem speziellen Elektrodensatz enthalten sind, fließen zu lassen,
**dadurch gekennzeichnet, dass**
die Steuereinheit (80) konfiguriert ist, die Schalteinheit (50) zu steuern, um in einem speziellen Zyklus während der Behandlung zwischen ersten Zuständen, bei denen der elektrische Hochfrequenzstrom zuerst in einen von mindestens zwei ersten Elektrodensätzen (201) und anschließend in einen anderen der mindestens zwei ersten Elektrodensätze (201) fließt, und einem zweiten Zustand, bei dem der elektrische Hochfrequenzstrom in einen zweiten Elektrodensatz (202) fließt, der die eine spezielle (Elektrode) der in dem einen der ersten Elektrodensätze (201) enthaltenen Elektroden (21, 22, 23) sowie die eine spezielle (Elektrode) der in dem anderen der ersten Elektrodensätze (201) enthaltenen Elektroden (21, 22, 23) umfasst, umzuschalten.

2. Vorrichtung zur Hochfrequenzbehandlung (2) gemäß Anspruch 1, wobei der zweite Elektrodensatz (202) nebeneinander angeordnete Elektroden umfasst.

3. Vorrichtung zur Hochfrequenzbehandlung (2) gemäß Anspruch 1 oder Anspruch 2, wobei die Elektroden (21, 22, 23) in das zu behandelnde Subjekt eingeführt werden.

4. Vorrichtung zur Hochfrequenzbehandlung (2) gemäß einem der Ansprüche 1 bis 3, wobei die Steuereinheit (80) zwischen dem ersten Zustand und dem zweiten Zustand in einem Zyklus von 0,3 Sekunden oder weniger umschaltet.

5. Vorrichtung zur Hochfrequenzbehandlung (2) gemäß einem der Ansprüche 1 bis 4, wobei die Ausgabeeinheit (40) für jeden der ersten Elektrodensätze (201) bereitgestellt ist.

## Revendications

1. Dispositif de traitement à haute fréquence (2) comprenant :
une unité de sortie (40) qui est configurée pour délivrer une puissance à haute fréquence ;
au moins trois électrodes (21, 22, 23) reliées à l'unité de sortie (40) et agencées dans un objet à traiter (100) ;
une unité de commutation (50) prévue entre l'unité de sortie (40) et les électrodes (21, 22, 23) pour commuter à un ensemble d'électrodes, parmi des ensembles électrodes comportant au moins trois des électrodes (21, 22, 23) combinées, dans lequel circule un courant électrique à haute fréquence ; et
une unité de commande (80) qui est configurée pour commander l'unité de commutation (50) pour amener le courant électrique à haute fréquence à circuler entre l'une spécifique des électrodes (21, 22, 23) incluse dans un premier ensemble d'électrodes (201) spécifique, dans lequel au moins trois des électrodes (21, 22, 23) sont combinées, et toutes les électrodes restantes incluses dans l'ensemble d'électrodes spécifique,
**caractérisé en ce que**
l'unité de commande (80) est configurée pour commander à l'unité de commutation (50) de commuter, dans un cycle spécifique au cours d'un traitement, entre des premiers états dans lesquels le courant électrique à haute fréquence circule d'abord dans l'un d'au moins deux premiers ensembles d'électrodes (201) puis dans un autre des au moins deux premiers ensembles d'électrodes (201), et un deuxième état dans lequel le courant électrique à haute fréquence circule dans un deuxième ensemble d'électrodes (202) incluant l'une spécifique des électrodes (21, 22, 23) incluse dans l'un des premiers ensembles d'électrodes (201) et l'une spécifique des électrodes (21, 22, 23) incluse dans l'autre des premiers ensembles d'électrodes (201).

2. Dispositif de traitement à haute fréquence (2) selon la revendication 1, dans lequel le deuxième ensemble d'électrodes (202) inclut des électrodes placées l'une à côté de l'autre.

3. Dispositif de traitement à haute fréquence selon la revendication 1 ou 2, dans lequel les électrodes (21, 22, 23) sont insérées dans l'objet à traiter (100).

4. Dispositif de traitement à haute fréquence (2) selon l'une quelconque des revendications 1 à 3, dans lequel l'unité de commande (80) commute entre le premier état et le deuxième état dans un cycle de 0,3 seconde ou moins.

5. Dispositif de traitement à haute fréquence (2) selon l'une quelconque des revendications 1 à 4, dans lequel l'unité de sortie (40) est prévue pour chacun des premiers ensembles d'électrodes (201).
